# EUROPÄISCHE PATENTSCHRIFT

(11) **EP 1 080 061 B1**
(45) Veröffentlichungstag und Bekanntmachung des Hinweises auf die Patenterteilung: **18.06.2003**
(21) Anmeldenummer: 99920726.9
(22) Anmeldetag: 21.04.1999
(51) Int. Cl.: C07C 31/20, C07C 29/136, C07C 29/88

(54) **VERFAHREN ZUR HERSTELLUNG VON GEMISCHEN AUS 1,4-BUTANDIOL, TETRAHYDROFURAN UND GAMMA-BUTYROLACTON**
METHOD FOR PRODUCING MIXTURES OF 1,4-BUTANEDIOL, TETRAHYDROFURAN AND GAMMA-BUTYROLACTONE
PROCEDE POUR LA PREPARATION DE MELANGES DE 1,4-BUTANDIOL, TETRAHYDROFURANE ET GAMMA-BUTYROLACTONE

(30) Priorität: 23.04.1998 DE 19818340
(43) Veröffentlichungstag der Anmeldung: 07.03.2001
(73) Patentinhaber: BASF AKTIENGESELLSCHAFT, 67056 Ludwigshafen (DE)
(72) Erfinder: FISCHER, Rolf, D-69121 Heidelberg (DE); KAIBEL, Gerd, D-68623 Lampertheim (DE); PINKOS, Rolf, D-67098 Bad Dürkheim (DE); RAHN, Ralf-Thomas, D-68167 Mannheim (DE)
(74) Vertreter: Isenbruck, Günter, Dr.
(86) Internationale Anmeldenummer: EP9902685
(87) Internationale Veröffentlichungsnummer: WO99055654

(56) Entgegenhaltungen:
- DE-A- 3 106 819

## Beschreibung

Die Erfindung betrifft ein Verfahren zur Herstellung von Gemischen enthaltend 1,4-Butandiol, Tetrahydrofuran (THF) und γ-Butyrolacton (GBL) aus dem Abgasstrom eines Reaktors zur Oxidation von Butan durch a) Absorption von Maleinsäureanhydrid (MSA) mit einem hochsiedenden Alkohol aus dem Abgasstrom, b) Umsetzung des dabei entstehenden Maleinsäurehalbesters zum Maleinsäurediester und c) dessen Hydrierung in der Flüssigphase.

Es sind zahlreiche Verfahren zur Umsetzung von MSA zu den entsprechenden Monound Diestern und dessen Hydrierung bekannt.

In EP-B 0 149 144, EP-B 0 206 194 und EP-B 0 212 121 werden Verfahren zur kontinuierlichen Abscheidung von MSA aus gasförmigen Reaktionsgemischen, die man bei der katalytischen Oxidation von Kohlenwasserstoffen erhält, beschrieben. Dabei wird das MSA enthaltende, gasförmige Reaktionsgemisch mit einem einwertigen Alkohol in Kontakt gebracht. Die dabei anfallenden gasförmigen Stoffe werden im Gegenstromverfahren mit Dicarbonsäurendiestern (EP-B 0 206 194 Fumarsäure- oder Bernsteinsäuredibutylester, EP-B 0 212 121 Fumarsäure-, Bernsteinsäure- oder Maleinsäurediester, EP-B 0 149 144 Maleinsäuredibutylester) in Berührung gebracht, und das flüssige Verfahrensprodukt wird dem Sumpf entnommen. Das flüssige Verfahrensprodukt enthält überwiegend die entsprechenden Mono- und Dialkylester der Maleinsäure. Diese werden zur Vervollstandigung der Veresterung auf 110°C bis 200°C erhitzt und sind danach ein geeignetes Edukt zur Hydrierung zu 1,4-Butandiol

Nachteilig bei diesem Verfahren ist, daß neben dem Alkohol noch ein zusätzlicher Stoff (Dicarbonsaurediester), der die gasförmigen, aus Alkohol und MSA entstehenden Reaktionsprodukte in die flüssige Phase überführt, im Kreis geführt wird. Dieser darf bei einer Hydrierung nicht mithydriert werden, so daß immer nur ein unvollständiger Umsatz erreicht werden kann was eine komplizierte Steuerung der Hydrierung bedeutet. Ferner muß bei einer destillativen Aufarbeitung der als Absorptionmittel eingesetzte Dicarbonsäuredibutylester aufwendig von Butandiol abgetrennt werden.

Auch DE-A 31 06 819 beschreibt ein Verfahren zur Herstellung von 1,4-Butandiol durch katalytische Hydrierung eines Gemisches, das man durch Behandlung von MSA enthaltenden gasförmigen Reaktionsgemischen mit aliphatischen Alkoholen erhält. Die Absorption des MSA erfolgt mit ein- oder zweiwertigen Alkoholen mit Siedepunkten über 180°C. Ein zusätzliches Absorptionsmittel ist nicht erforderlich. Die anschließende Nachveresterung des Absorptionsaustrages erfolgt bei 120°C bis 150°C. Der diesterhaltige Strom wird abschließend katalytisch hydriert, wobei die Raum-Zeit-Ausbeute (0,04 kg Butandiol/liter x Stunde) gering ist, da die Säurezahl nach der Nachveresterung zu hoch ist.

Die oben genannten Verfahren sind technisch durchführbar, haben jedoch Nachteile, die zu hohen Herstellkosten führen. Diese Nachteile sind insbesondere eine komplizierte Steuerung der Hydrierung beziehungsweise eine geringe Raum-Zeit-Ausbeute bei der katalytischen Hydrierung.

Aufgabe der vorliegenden Erfindung ist es daher, ein Verfahren bereitzustellen, das möglichst wenig Absorptionsmittel benötigt, bei der katalytischen Hydrierung eine gute Raum-Zeit-Ausbeute mit preiswerten Katalysatoren erreicht und bei der Trennung der Wertprodukte keine aufwendig zu trennenden Stoffgemische bildet.

Diese Aufgabe wird gelöst durch ein Verfahren zur Herstellung von 1,4-Butandiol, Tetrahydrofuran (THF) und γ-Butyrolacton (GBL) durch Oxidation von Butan zu einem Maleinsäureanhydrid enthaltenden Produktstrom, Absorption von Maleinsäureanhydrid aus dem Produktstrom mit einem hochsiedenden Alkohol, wobei ein flüssiger Absorptionsaustrag erhalten wird, der Maleinsäurehalb- und -diester sowie hochsiedenden Alkohol enthält, Nachveresterung des flüssigen Absorptionsaustrages und anschließende Hydrierung des nachveresterten Austrages in der Flüssigphase. Das erfindungsgemäße Verfahren ist dann dadurch gekennzeichnet, daß der hochsiedende Alkohol ein mehrwertiger Alkohol mit einem Siedepunkt bei Normaldruck von über 233°C ist und die Nachveresterung bei einer Verweilzeit von maximal 3 strung und Temperaturen von 160-300°C durchgeführt wird.

Der nachveresterte Austragweist eine Säurezahl von unter 30 mg KOH/g und einen Wassergehalt von unter 1 Gew.-% auf.

Im Kontext der vorliegenden Erfindung ist unter Absorption die Abscheidung von MSA aus einem durch Oxidation von Butan erhaltenen Produktstrom mit einem hochsiedenden Alkohol zu verstehen. Dabei findet eine Reaktion des MSA mit dem Alkohol zu einem Maleinsäurehalbester statt, der das Hauptprodukt des Absorptionsaustrages bildet.

In diesem Verfahren wird durch Zugabe des Veresterungsalkohols bei den herrschenden Reaktionsbedingungen direkt ein flüssiges Verfahrensprodukt erhalten. Der zusätzliche Einsatz eines hochsiedenden Absorptionsmittels wie Dicarbonsäurediester ist nicht notwendig. Dadurch wird eine aufwendige spätere Abtrennung der Wertprodukte vermieden. Die Hydrierung kann aufgrund der niedrigen Säurezahl mit preiswerten Katalysatoren und in guten Ausbeuten mit dem nachveresterten Produkt durchgeführt werden. Dadurch können Kosten eingespart werden.

Durch katalytische Oxidation von Butan oder eines anderen Kohlenwasserstoffs in der Gasphase an einem mit z.B. MoO₃ aktivierten Vanadium-Pentoxid-Katalysator erhält man einen MSA und Nebenprodukte wie Kohlendioxid, Essigsäure und Acrylsäure enthaltenden Produktstrom. Im erfindungsgemäßen Verfahren bringt man diesen MSAenthaltenden, gasförmigen Produktstrom zum Zwecke der MSA-Absorption mit einem hochsiedendem Alkohol in Kontakt. Die Reaktionsgaszufuhr erfolgt bei einer zweckmäßigen Ausführungsform des Verfahrens unterhalb der Oberfläche des flüssigen, hochsiedendem Alkohols, z.B. durch ein Tauchrohr. Bevorzugt ist ein Verfahren, in dem das Reaktionsgemisch direkt von unten in einer Absorptionskolonne eingeleitet wird, wo ihm der flüssige, hochsiedende Alkohol entgegenströmt. Die Arbeitsweise, bei der man die MSA-Absorption in einer Kolonne oder mehreren hintereinander geschalteten Kolonnen durchführt, ist bevorzugt.

Als Kolonnen kommen z.B. Glockenbodenkolonnen, Packungskolonnen oder Füllkörperkolonnen in Frage, dabei werden Füllkörperkolonnen bevorzugt eingesetzt. Diese können mit Zwischenkühlern versehen werden, um die Absorptionswärme abzuführen.

Der Gehalt an MSA im Produktstrom der Butanoxidation ist für das erfindungsgemäße Verfahren nicht kritisch. Bei gängigen Verfahren liegt er im Bereich von 0,5 bis 2 Vol.-%. Auch die Temperatur des Abgasstroms ist im allgemeinen nicht kritisch. Diese sollte, um Verbackungen zu vermeiden, möglichst nicht unterhalb der Tau- und Schmelztemperaturen der einzelnen Komponenten liegen, also vorzugsweise mindestens 100°C betragen.

Die bei der Oxidation des Butans auftretenden Nebenprodukte wie CO_{2,} Essigsäure, Acrylsäure sind für das Verfahren ebenfalls nicht kritisch. Sie werden entweder überwiegend mit dem Abgasstrom mitgerissen oder bei der Absorption gebunden. Sofern sie nicht mit dem Veresterungsalkohol reagieren, werden sie bei der thermischen Behandlung des flüssigen Absorptionsaustrages (zum Teil noch mit dem Wasser) entfernt.

Als Alkohole werden im erfindungsgemäßen Verfahren mehrwertige Alkohole mit einem Siedepunkt bei Normaldruck von über 233°C, bevorzugt von über 250°C eingesetzt. Als mehrwertige Alkohole werden dabei bevorzugt 2- bis 4-wertige Alkohole, besonders bevorzugt 2-wertige Alkohle (Diole) eingesetzt. Beispielsweise werden Polyethylenglykole, α,ω-Diole und Cyclohexandimethanole, wie 1,5-Pentandiol, 1,6-Hexandiol, 1,7-Heptandiol, 1,8-Octandiol, 1,12-Dodecandiol, 1,4-Cyclohexandimethanol, 1,3-Cyclohexandimethanol, Trimethylolpropan, Neopentylglykol, Triethylenglykol, Tetraethylenglykol, Pentaethylenglykol, ganz besonders bevorzugt 1,6-Hexandiol und 1,4-Cyclohexan-dimethanol eingesetzt. Als 3-wertiger Alkohol kann Glycerin, als 4-wertiger Alkohol Pentaerythrit zum Einsatz kommen. Die Alkohole können in reiner Form oder als Gemische verschiedener Alkohole eingesetzt werden.

Im allgemeinen wird der Alkohol im Verhältnis zu MSA in einem bis zu 30-molaren Überschuß, bevorzugt in einem bis zu 15-molaren Überschuß, ganz besonders bevorzugt in einem bis zu 5-molaren Überschuß eingesetzt.

Bei der Absorption entsteht aus dem MSA und dem Alkohol zuerst ein Maleinsäurehalbester. Dieser ist schwersiedend und bei den Reaktionstemperaturen somit nicht mehr flüchtig. Die Absorption kann so gestaltet werden, daß in der zur Absorption von MSA verwendeten Apparatur bereits die Nachveresterung des Halbesters zum Diester erfolgt. Dabei kann der Halbester mit weiterem freien Alkohol oder mit einem anderen Halbester reagieren. Das bei der Reaktion abgespaltene Wasser kann, sofern die Temperaturen hoch genug sind, mit dem Abgasstrom entfernt werden.

Enthält der flüssige Austrag noch größere Mengen Halbester, so ist es bevorzugt, eine thermische Nachveresterung in einem Verweilzeitgefäß durchzuführen und so die Veresterung zum Diester zu vervollständigen. Die Nachveresterung kann mit oder ohne Zugabe eines Veresterungskatalysators erfolgen.

Als Veresterungskatalysatoren können prinzipiell alle zur Veresterung von Säuren bekannten homogenen oder heterogenen Katalysatoren eingesetzt werden. Dabei sind heterogene Katalysatoren wie TiO₂, ZrO₂, Al₂O₃, SiO₂, Silikate, Zeolithe, Heteropolysäuren, oder saure Ionenaustauscher bevorzugt.

Besonders bevorzugt wird ohne Katalysator, rein thermisch, verestert. Der Monoester enthaltende Strom wird bei Temperaturen von 160°C bis 300°C, bevorzugt 160°C bis 250°C, besonders bevorzugt 180°C bis 240°C nachverestert. Das Reaktionswasser wird im allgemeinen kontinuierlich destillativ entfernt. Die Verweilzeit beträgt üblicherweise maximal 3 Stunden, im allgemeinen zwischen 0,1 und 3 Stunden, bevorzugt 0,2 und 2,5 Stunden, besonders bevorzugt zwischen 0,3 und 2 Stunden. Zur Abtrennung des Reaktionswassers ist es auch möglich, die Nachveresterung in einer Strippkolonne durchzuführen und das als Wasserdampf freiwerdende Wasser durch Strippgas zu entfernen. Es ist weiterhin möglich, Vakuum anzulegen, um das Reaktionswasser leichter abzutrennen. Ferner ist es möglich, Schleppmittel für Wasser, wie aromatische Kohlenwasserstoffe, zuzusetzen. Der Wassergehalt nach der Veresterung liegt unter 1 Gew.-%, bevorzugt unter 0,5%, besonders bevorzugt unter 0,2%.

Aufgrund der Wahl der Veresterungsbedingungen ist der Gehalt an freien Säuren im Veresterungsaustrag gering. Der Gehalt an freien Säuren (gemessen durch Titration) liegt vor der Hydrierung unter 30 mg KOH/g, bevorzugt unter 20 mg KOH/g, besonders bevorzugt unter 10 mg KOH/g.

Die Hydrierung kann in Gegenwart eines inerten organischen Lösungsmittels erfolgen, insbesondere wenn nur kleine Mengen hydriert werden sollen. Beispielsweise kann der Veresterungsaustrag zur besseren Pumpbarkeit mit einem inerten organischen Lösungsmittel, bevorzugt mit Ethylenglykoldimethylether, verdünnt werden. Bei der technischen Umsetzung verzichtet man im allgemeinen auf ein zusätzliches Lösungsmittel.

Die Hydrierung erfolgt diskontinuierlich oder kontinuierlich in der Flüssigphase an fest angeordneten oder suspendierten oder homogen, löslichen Katalysatoren. Bevorzugt ist eine kontinuierliche Durchführung. Bei fest angeordneten Katalysatoren kann in Rieseloder Sumpffahrweise, mit oder ohne Produktrückführung hydriert werden. Es können ein oder mehrere Reaktoren hintereinander oder parallel betrieben werden. Beispielsweise kann so gearbeitet werden, daß in einem ersten Reaktor überwiegend die C-C-Doppelbindung der Maleinsäureester hydriert wird, wobei Bernsteinsäureester entstehen und anschließend in einem zweiten Reaktor zu Butandiol, THF und GBL weiterhydriert wird

Es wird bei Temperaturen im allgemeinen zwischen 70°C und 350°C, bevorzugt zwischen 80°C und 300°C, besonders bevorzugt zwischen 80°C und 260°C und bei Drucken im allgemeinen zwischen 20 bar und 350 bar, bevorzugt zwischen 40 bar und 320 bar, besonders bevorzugt zwischen 60 bar und 300 bar hydriert.
Als Hydrierkatalysatoren können im erfindungsgemäßen Verfahren im allgemeinen heterogene oder homogene zur Hydrierung von Carbonylgruppen geeignete Katalysatoren eingesetzt werden. Bevorzugt sind heterogene Katalysatoren. Beispiele hierfür sind in Houben-Weyl, Methoden der Organischen Chemie, Band IV/1c, S. 16 bis 26, Georg Thieme Verlag, 1980 beschrieben.

Von diesen Hydrierkatalysatoren sind solche bevorzugt, die mindestens ein Element der Gruppe Ib, VIb, VIIb und VIIIb, sowie IIIa, IVa und Va des Periodensystems der Elemente, insbesondere Kupfer, Chrom, Rhenium, Kobalt, Rhodium, Nickel, Palladium, Eisen, Platin, Indium, Zinn und Antimon enthalten. Besonders bevorzugt sind Katalysatoren, die Kupfer, Kobalt, Palladium, Platin oder Rhenium enthalten. Aus dieser Gruppe sind solche ganz besonders bevorzugt, die Kupfer enthalten.

Ein Beispiel für die Art der Katalysatoren, die für das erfindungsgemäße Verfahren eingesetzt werden können, sind sog. Vollkatalysatoren. Dabei sind die katalytisch wirkenden Metalle ganz überwiegend ohne Trägermaterialien vorhanden. Beispiele hierzu sind die sog. Raney-Katalysatoren, z.B. auf Basis Ni, Cu oder Kobalt. Andere Beispiele sind Pd-Schwarz, Pt-Schwarz, Cu-Schwamm, Legierungen oder Mischungen aus z.B. Pd/Re, Pt/Re, Pd/Ni, Pd/Co oder Pd/Re/Ag.
Die im erfindungsgemäßen Verfahren eingesetzten Katalysatoren können auch sogenannte Fällungskatalysatoren sein. Solche Katalysatoren können hergestellt werden, indem man ihre katalytisch aktiven Komponenten aus deren Salzlösungen, insbesondere aus deren Nitrat- und/oder Acetatlösungen, beispielsweise durch Zugabe von Alkalimetall- und/oder Erdalkalimetallhydroxid- und/oder Carbonat-Lösungen, z.B. als schwerlösliche Hydroxyde, Oxydhydrate, basische Salze oder Carbonate ausfällt. Die erhaltenen Niederschläge werden anschließend getrocknet und dann durch Calcinierung bei im allgemeinen 300 bis 700 °C, bevorzugt 400 bis 600 °C, in die entsprechenden Oxide, Mischoxide und/oder gemischtvalentigen Oxide umgewandelt. Diese werden durch eine Behandlung mit Wasserstoff oder mit Wasserstoff enthaltenden Gasen bei in der Regel 50 bis 700 °C, bevorzugt 100 bis 400 °C zu den betreffenden Metallen und/oder oxidischen Verbindungen niederer Oxidationsstufe reduziert und in die eigentliche, katalytisch aktive Form überführt. Dabei wird in der Regel so lange reduziert, bis kein Wasser mehr gebildet wird.

Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können bevorzugt auch gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den oben genannten Fällungskatalysatoren, die außer den katalytisch aktiven Komponenten zusätzlich ein Trägermaterial enthalten, eignen sich für das erfindungsgemäße Verfahren im allgemeinen solche Trägermaterialien, bei denen die katalytisch-hydrierend wirkenden Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedene Art und Weise durchgeführt werden. Die katalytisch aktiven Metalle können auf diesen Trägermaterialien z.B. durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, bevorzugt mit Wasserstoff oder komplexen Hydriden, aufgebracht werden.

Eine andere Moglichkeit zur Aufbringung der katalytisch aktiven Metalle auf diese Trager besteht darin, die Träger mit Losungen thermisch leicht zersetzbarer Salze, z.B. mit Nitraten oder thermisch leicht zersetzbaren Komplexverbindungen, z.B Carbonyloder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600 °C zu erhitzen. Diese thermische Zersetzung wird bevorzugt unter einer Schutzgasatmosphäre vorgenommen. Geeignete Schutzgase sind z.B. Stickstoff, Kohlendioxid, Wasserstoff oder die Edelgase.

Weiterhin können die katalytisch aktiven Metalle auf dem Katalysatorträger durch Aufdampfen oder durch Flammspritzen abgeschieden werden.

Der Gehalt der katalytisch aktiven Metalle an den Trägerkatalysatoren ist prinzipiell für das Gelingen des erfindungsgemäßen Verfahrens nicht kritisch. Höhere Gehalte an katalytisch aktiven Metallen führen diese Trägerkatalysatoren im allgemeinen zu höheren Raum-Zeit-Umsätzen als niedrigere Gehalte.

Im allgemeinen werden Trägerkatalysatoren verwendet, deren Gehalt an katalytisch aktiven Metallen 0,1 bis 90 Gew.-%, bevorzugt 0,5 bis 40 Gew.-%, bezogen auf den gesamten Katalysator, beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindungsgemäßen Verfahrens auswirkt.
Es können auch mehrere der katalytisch aktiven Metalle auf dem jeweiligen Trägermaterial aufgebracht sein.

Weiterhin können die katalytisch aktiven Metalle beispielsweise nach den in DE-A 25 19 817, EP-A 0 147 219 und EP-A 0 285 420 beschriebenen Verfahren auf den Träger aufgebracht werden. In den Katalysatoren gemäß den vorgenannten Schriften liegen die katalytisch aktiven Metalle als Legierung vor. Diese werden der z.B. durch Tränkung mit einem Salz oder Komplex der zuvor genannten Metalle und anschließende thermische Behandlung und/oder Reduktion erzeugt

Sowohl die Aktivierung der Fällungskatalysatoren als auch der Trägerkatalysatoren kann auch in situ zu Beginn der Reaktion durch den anwesenden Wasserstoff erfolgen, bevorzugt werden diese Katalysatoren jedoch vor ihrer Verwendung separat aktiviert.

Als Trägermaterialien können im allgemeinen die Oxide des Aluminiums und Titans, Zirkoniumdioxid, Siliciumdioxid, Tonerden, z.B. Montmorillonite, Silikate wie Magnesiumoder Aluminiumsilikate, Zeolithe wie ZSM-5 -oder ZSM-10-Zeolithe, sowie Aktivkohle verwendet werden. Bevorzugte Trägermaterialien sind Aluminiumoxide, Titandioxide, Siliciumdioxid, Zirkoniumdioxid und Aktivkohle. Es können auch Mischungen verschiedener Trägermaterialien als Träger für die im erfindungsgemäßen Verfahren anwendbaren Katalysatoren dienen.

Für im erfindungsgemäßen Verfahren einsetzbare Heterogenkatalysatoren seien die folgenden beispielhaft genannt: Kobalt auf Aktivkohle, Kobalt auf Siliciumdioxid, Kobalt auf Aluminiumoxid, Rhenium auf Aktivkohle, Rhenium auf Siliciumdioxid, Rhenium/Zinn auf Aktivkohle, Rhenium/Platin auf Aktivkohle, Kupfer auf Aktivkohle, Kupfer/Siliciumdioxid, Kupfer/Aluminiumoxid, Kupferchromit, Bariumkupferchromit, Kupfer/Aluminiumoxid/Manganoxid, Kupfer/Aluminiumoxid/Zinkoxid sowie die Katalysatoren gemäß DE-A 39 32 332, US-A 3 449 445, EP-A 0 044 444, EP-A 0 147 219, DE-A 39 04 083, DE-A 23 21 101, EP-A 0 415 202, DE-A 23 66 264, EP-A 0 552 463 und EP-A 0 100 406.

Bevorzugte Katalysatoren enthalten mindestens eines der Metalle Kupfer, Mangan, Rhenium, Kobalt, Chrom, Palladium, Platin oder Nickel. Besonders bevorzugt sind Kupfer, Kobalt, Palladium, Platin oder Rhenium.

Der Gehalt an den einzelnen Wertprodukten im Hydrieraustrag 1,4-Butandiol, THF und GBL zueinander kann schwanken. Beispielhaft sei folgendes molare Verhältnis der Produkte zueinander genannt, wobei die Summe der molaren Anteile von 1,4-Butandiol, THF und GBL 100 mol-% beträgt: Butandiol 50 bis 95 mol-%, THF 2 bis 40 mol-%, GBL 0,1 bis 20 mol.-%. Das Verhaltnis der Wertprodukte zueinander wird vorwiegend bei der Hydrierung durch die Parameter Druck, Temperatur, Verweilzeit und Katalysator bestimmt. So kann der Gehalt an GBL beispielsweise fast auf 0 abgesenkt werden, wenn bei hohem Druck, niedriger Temperatur und langer Verweilzeit hydriert wird. Der Gehalt an THF kann hoch sein, wenn der Hydrierkatalysator saure Zentren aufweist.

Weitere Produkte, die im Hydrieraustrag enthalten sein können bzw. enhalten sind, sind beispielsweise Wasser, n-Butanol, n-Propanol und Bernsteinsäureester sowie der Absorptionsalkohol. Der Bernsteinsäureester kann dabei als Alkoholkomponente sowohl den zuvor eingesetzten Absorptionsalkohol als auch auch Butandiol enthalten. Der Bernsteinsäureester kann zusammen mit dem Absorptionsalkohol zurückgeführt werden.

Die Aufarbeitung der Reaktionsprodukte kann in einer für den Fachmann geläufigen Weise erfolgen. Bevorzugt ist eine destillative Aufarbeitung. Dabei kann beispielsweise so vorgegangen werden, daß zuerst Leichtsieder wie THF und eventuell vorhandenes Wasser, Butanol oder Propanol über dem Kopf einer Destillationskolonne, und die verbleibenden Produkte Butandiol und GBL dann vom zurückbleibenden Sumpfstrom abdestilliert werden. Der resultierende Sumpfstrom der ganz überwiegend den Absorptionsalkohol enthält, wird anschließend wieder in die MSA-Absorption zurückgeführt. Gegebenenfalls kann dabei ein kleiner Ausschleusestrom abgetrennt werden.

Die folgenden Beispiele erläutern die Erfindung zusätzlich.

### Beispiel 1:

Ein Abgasstrom einer n-Butanoxidation, der aus ca. 1 Vol.-% MSA und 99 Vol.-% Luft bestand, wurde mit einer Temperatur von 100°C in den unteren Teil einer Füllkörperkolonne geleitet, die ca. 25 theoretische Trennstufen aufwies und mit Zwischenkühlern versehen war, um die Absorptionswärme abzuführen. Die Zwischenkühler wurden auf einer Temperatur von ca. 65°C gehalten. Auf den Kopf der Kolonne wurde 70°C warmes 1,4-Cyclohexandimethanol gepumpt. Am Kopf der Kolonne stellte sich eine Temperatur von 87°C ein, am Sumpf der Kolonne eine Temperatur von 105°C.
Insgesamt wurden ca. 25 g MSA durch 144 g 1,4-Cyclohexandimethanol gebunden. Der Austrag der Absorption wurde anschließend in einem Verweilzeitgefäß 2,5 h auf 200°C und noch 30 Minuten auf 225°C erhitzt. Dabei wurde das Veresterungswasser abdestilliert. Der Austrag enthielt danach 0,19 Gew.-% Wasser und die Säurezahl lag bei 19,1 mg KOH/g. Der Austrag wurde zur besseren Pumpbarkeit mit der gleichen Menge Ethylenglycoldimethylether verdünnt und an 25 ml eines Cu-Katalysators der Süd-Chemie AG-München (T 4489) bei 220°C und 220 bar kontinuierlich hydriert (ca. 20 g Zulauf/h, Rohrreaktor, Rieselfahrweise ohne Produktrückführung). Im farblosen Hydrieraustrag fanden sich - Cyclohexandimethanol und Ethylenglycoldimethylether herausgerechnet - 70 mol-% 1,4-Butandiol, 10 mol-% THF und unter 1 mol-% GBL. Der Rest bestand überwiegend aus n-Butanol und Bernsteinsäurediester. Der Austrag wurde destillativ aufgearbeitet, wobei THF, Butanol, Ethylenglycoldimethylether, GBL und 1,4-Butandiol abdestilliert wurden. Im Sumpf verblieben überwiegend Cyclohexandimethanol und Bernsteinsäurediester.

### Beispiel 2:

Analog Beispiel 1 wurde MSA mit 1,6-Hexandiol absorbiert. Dabei wurden 73,5 g MSA mit 177 g Hexandiol umgesetzt. Die Nachveresterung wurde 1 h bei 200°C und 225°C vorgenommen. Der Veresterungsaustrag enthielt 0,27 Gew.-% Wasser und wies eine Säurezahl von 8,3 mg KOH/g auf. Vor der Hydrierung wurde wie in Beispiel 1 mit Ethylenglycoldimethylether verdünnt und wie in Beispiel 1 hydriert, allerdings diesmal bei 220 bar bzw. 110 bar. Bei 220 bar fanden sich im farblosen Hydrieraustrag - Hexandiol und Ethylenglycoldimethylether herausgerechnet - 85 mol-% 1,4-Butandiol, 3 mol-% THF und ca. 0,2 mol-% GBL. Der Rest bestand überwiegend aus Butanol und Bernsteinsäurediester.
Bei 110 bar fanden sich im farblosen Hydrieraustrag - Hexandiol und Ethylenglycoldimethylether herausgerechnet - 68 mol-% 1,4-Butandiol, 2 mol-% THF und 8 mol-% GBL. Der Rest bestand überwiegend aus Butanol und Bernsteinsäurediester.

### Vergleichsbeispiel V1 (DE-A 31 06 819)

Analog Beispiel 2 wurde MSA mit Hexandiol umgesetzt, die Veresterung aber 3 h bei 150°C durchgeführt. Der Veresterungsaustrag wies einen Wassergehalt von 0,2 % und eine Säurezahl von 49 mg KOH/g auf. Die Hydrierung wurde bei 110 bar wie in Beispiel 2 durchgeführt. Bereits nach kurzer Zeit verfärbte sich der zuvor farblose Hydrieraustrag (Kupfer/Mangan) rotbraun und die Hydrieraktivität sank ab.

## Patentansprüche

1. Verfahren zur Herstellung von 1,4-Butandiol, Tetrahydrofuran und γ-Butyrolacton durch Oxidation von Butan zu einem Maleinsäureanhydrid enthaltenden Produktstrom, Absorption von Maleinsäureanhydrid aus dem Produktstrom mit einem hochsiedenden Alkohol, wobei ein flüssiger Absorptionsaustrag erhalten wird, der Maleinsäurehalb- und -diester sowie hochsiedenden Alkohol enthält, Nachveresterung des flüssigen Absorptionsaustrages und anschließende Hydrierung des nachveresterten Austrages in der Flüssigphase, **dadurch gekennzeichnet, daß** der hochsiedende Alkohol ein mehrwertiger Alkohol mit einem Siedepunkt bei Normaldruck von über 233°C ist und 1 die Nachveresterungbei einer Verweilzeit von maximal 3 Stunden und Temperaturen von 160 bis 300°C durchgeführt wird.

2. Verfahren nach Anspruch 1, **dadurch gekennzeichnet, daß** als Alkohol 1,6-Hexandiol oder 1,4-Cyclohexandimethanol eingesetzt wird.

3. Verfahren nach Anspruch 1 oder 2, **dadurch gekennzeichnet, daß** die Nachveresterung in der zur Absorption von Maleinsäureanhydrid verwendeten Apparatur durchgeführt wird.

4. Verfahren nach einem der Ansprüche 1 bis 3, **dadurch gekennzeichnet, daß** die Hydrierung bei Temperaturen zwischen 70°C und 350°C und bei einem Druck von 20 bis 350 bar durchgeführt wird.

5. Verfahren nach einem der Ansprüche 1 bis 4, **dadurch gekennzeichnet, daß** die Hydrierung in Gegenwart eines Katalysators durchgeführt wird, der mindestens ein Element der Gruppe Ib, VIb, VIIb, VIIIb des Periodensystems der Elemente enthält.

6. Verfahren nach Anspruch 5, **dadurch gekennzeichnet, daß** der Hydrierkatalysator Kupfer enthält.

7. Verfahren nach einem der Ansprüche 1 bis 6, **dadurch gekennzeichnet, daß** der Gehalt der einzelnen Wertprodukte im Hydrieraustrag 1,4-Butandiol, Tetrahydrofuran und γ-Butyrolacton zueinander 50 bis 95 mol-% 1,4-Butandiol, 2 bis 40 mol-% Tetrahydrofuran und 0,1 bis 20 mol-% γ-Butyrolacton beträgt, wobei die Summe der molaren Anteile von 1,4-Butandiol, Tetrahydrofuran und γ-Butyrolacton 100 mol-% beträgt.

8. Verfahren nach einem der Ansprüche 1 bis 7, **dadurch gekennzeichnet, daß** der hochsiedende Alkohol nach der Hydrierung wieder zur Absorption zurückgeführt wird.

## Claims

1. A process for preparing 1,4-butanediol, tetrahydrofuran and γ-butyrolactone by oxidation of butane to give a product stream comprising maleic anhydride, absorption of maleic anhydride from the product stream using a high-boiling alcohol to give a liquid absorption product comprising monoesters and diesters of maleic acid and also high-boiling alcohol, after-esterification of the liquid absorption product and subsequent hydrogenation of the after-esterified product in the liquid phase, wherein the high-boiling alcohol is a polyhydric alcohol having a boiling point at atmospheric pressure of above 233°C and the after-esterification is carried out a residence time of not more than 3 hours and at from 160 to 300°C.

2. A process as claimed in claim 1, wherein the alcohol used is 1,6-hexanediol or 1,4-cyclohexanedimethanol.

3. A process as claimed in claim 1 or 2, wherein the after-esterification is carried out in the apparatus used for the absorption of maleic anhydride.

4. A process as claimed in any of claims 1 to 3, wherein the hydrogenation is carried out at from 70°C to 350°C and at a pressure of from 20 to 350 bar.

5. A process as claimed in any of claims 1 to 4, wherein the hydrogenation is carried out in the presence of a catalyst comprising at least one element of groups Ib, VIb, VIIb and VIIIb of the Periodic Table of the Elements.

6. A process as claimed in claim 5, wherein the hydrogenation catalyst comprises copper.

7. A process as claimed in any of claims 1 to 6, wherein the ratio of the individual desired products in the hydrogenation product, viz. 1,4-butanediol, tetrahydrofuran and γ-butyrolactone, is 50-95 mol% of 1,4-butanediol, 2-40 mol% of tetrahydrofuran and 0.1-20 mol% of γ-butyrolactone, where the sum of the mole fractions of 1,4-butanediol, tetrahydrofuran and γ-butyrolactone is 100 mol%.

8. A process as claimed in any of claims 1 to 7, wherein the high-boiling alcohol is returned to the absorption after the hydrogenation.

## Revendications

1. Procédé de fabrication du 1,4-butandiol, tétrahydrofurane et γ-butyrolactone par oxydation du butane en un flux de produit contenant l'anhydride maléique, absorption de l'anhydride maléique du flux de produit avec un alcool à ébullition élevée, dans lequel un extrait liquide d'absorption qui contient le mono et le diester maléique ainsi que l'alcool à ébullition élevée est obtenu, désestérification de l'extrait liquide d'absorption et hydratation liante de l'extrait désestérifié dans la phase liquide, **caractérisé en ce que** l'alcool à ébullition élevée est un alcool plurivalent avec un point d'ébullition de plus de 233 °C sous pression normale et la désestérification est menée avec un temps de séjour maximal de 3 heures à des températures de 160 °C à 300 °C.

2. Procédé selon la revendication 1, **caractérisé en ce que** le 1,6-hexanediol ou le 1,4-cyclohexanedimethanol est mis en place pour l'alcool.

3. Procédé selon la revendication 1 ou 2, **caractérisé en ce que** la désestérification est menée dans l'appareillage utilisé pour l'absorption de l'anhydride maléique.

4. Procédé selon l'une quelconque des revendications 1 à 3, **caractérisé en ce que** l'hydratation est menée à une température entre 70 °C et 350 °C et à une pression de 20 à 350 bars.

5. Procédé selon l'une quelconque des revendications 1 à 4, **caractérisé en ce que** l'hydratation est menée en présence d'un catalyseur, qui comprend au moins un élément des groupes Ib, VIb, VIIb, VIIIb de la classification périodique des éléments.

6. Procédé selon la revendication 5, **caractérisé en ce que** le catalyseur de l'hydratation contient du cuivre.

7. Procédé selon l'une quelconque des revendications 1 à 6, **caractérisé en ce que** la teneur des uniques produits de valeur 1,4-butandiol, tétrahydrofurane et γ-butyrolactone dans l'extrait d'hydratation s'élèvent les uns par rapport aux autres de 50 à 95 % en moles de 1,4-butanediol, 2 à 40 % en moles de tétrahydrofurane et 0,1 à 20 % en moles de γ-butyrolactone, moyennant quoi la somme des fractions molaires du 1,4-butandiol, tétrahydrofurane et γ-butyrolactone s'élève à 100 % en moles.

8. Procédé selon l'une quelconque des revendications 1 à 7, **caractérisé en ce que** l'alcool à ébullition élevée est redirigé vers l'absorption après l'hydratation.
